# EUROPEAN PATENT SPECIFICATION

(11) **EP 4 277 554 B1**
(45) Date of publication and mention of the grant of the patent: **28.10.2026**
(21) Application number: 21919211.9
(22) Date of filing: 14.01.2021
(51) Int. Cl.: A61B 18/14, A61M 29/02, A61B 5/283, A61B 18/00, A61B 5/00

(54) **MEDICAL DILATOR**
MEDIZINISCHER DILATATOR
DILATATEUR MÉDICAL

(43) Date of publication of application: 22.11.2023
(60) Divisional application: 26206104.7
(73) Proprietor: Boston Scientific Medical Device Limited, Galway H91 Y868 (IE)
(72) Inventor: DAVIES, Gareth, Mississauga, Ontario L4W 5P6 (CA); KOON, Lauren, Mississauga, Ontario L4W 5P6 (CA); URBANSKI, John Paul, Mississauga, Ontario L4W 5P6 (CA); MORIYAMA, Eduardo, Mississauga, Ontario L4W 5P6 (CA)
(74) Representative: Pfenning, Meinig & Partner mbB
(86) International application number: PCT/IB2021/050266
(87) International publication number: WO 2022/153082

(56) References cited:
- EP-B1- 2 996 755
- WO-A1-2018/165277
- US-A1- 2003 060 822
- US-A1- 2006 089 691
- US-A1- 2014 206 987
- US-A1- 2015 250 424
- US-A1- 2017 079 541
- US-A1- 2020 060 710
- US-B1- 8 019 401
- US-B2- 10 493 259

## Description

### FIELD:

This document relates to medical dilation, for example dilation of a surgically created perforation in cardiac tissue. More specifically, this document relates to a medical dilator, and related systems, methods, and kits.

Document US 2003/060822 A1 describes a tissue ablation device assembly which ablates a region of tissue in a body of a patient. The tissue ablation device assembly comprises an elongated body having a proximal end portion and a distal end portion. A tubular porous membrane having a porous wall with an inner surface that defines an inner space is located along the distal end portion of the elongated body. An ablation element is disposed over the porous membrane, with the ablation element having a fixed position with respect to the porous membrane. A fluid passageway extending through the elongated body and communicates with the inner space. The fluid passageway is adapted to be fluidly coupled to a pressurizeable fluid source for delivering a volume of pressurized fluid from the fluid source to the inner space. The porous membrane allows at least a substantial portion of the volume of pressurized fluid to pass through the porous wall for enhancing ablative coupling between the electrode and the region of tissue.

Document WO 2018/165277 A1 describes a prior art medical dilator having a lumen, an electrode and a tapered tip.

### SUMMARY:

The following summary is intended to introduce the reader to various aspects of the detailed description, but not to define or delimit any invention. The invention is set out in the appended set of claims and all examples and methods are to be understood as examples useful for understanding the invention and not as embodiments of the invention as claimed, if they are not within the scope of the claims.

According to the invention, a medical dilator includes an elongate member having a proximal end portion, an opposed distal end portion, and a lumen extending through the elongate member from the proximal end portion to the distal end portion. A dilating tip is at the distal end portion. The dilating tip has a first end of enlarged cross-sectional area and tapers going in the distal direction to a second end of reduced cross-sectional area. At least a first electrode is associated with the dilating tip. An electrical conductor is electrically connected to the first electrode and extends proximally from the first electrode towards the proximal end portion for electrical connection with an electroanatomical mapping system.

In some examples, the first electrode is positioned between the first end of the dilating tip and the second end of the dilating tip. In some examples, the first electrode is positioned proximal of the first end of the dilating tip.

In some examples, the dilating tip has a tip circumferential outer surface having a circumferential groove defined therein, and the electrode is annular and is seated in the groove.

In some examples, the dilating tip has a tip circumferential outer surface, a tip circumferential inner surface, and a tip sidewall extending between the tip circumferential inner surface and the tip circumferential outer surface, and the electrical conductor extends from the electrode through the tip sidewall and into the lumen.

In some examples, the elongate member has a circumferential outer surface, a circumferential inner surface, and a sidewall extending along the length of the elongate member between the circumferential inner surface and the circumferential outer surface, and the electrical conductor is embedded in the sidewall and extends from the electrode to the proximal end portion. The circumferential outer surface can have a longitudinal groove defined therein and extending from the first electrode to the proximal end portion, and the electrical conductor can be seated in the longitudinal groove. Alternatively, the elongate member can include an outer tube defining the circumferential outer surface, and an inner liner within the outer tube and defining the circumferential inner surface, and the electrical conductor can be positioned between the outer tube and the inner liner. The electrical conductor can be a tubular braid.

In some examples, the first electrode is removable from the elongate member.

In some examples, the medical dilator further includes a second electrode mounted to the elongate member and spaced from the first electrode.

In accordance with the invention, the dilating tip includes a proximal piece having a distal-facing shoulder surface and a neck extending distally from the shoulder surface, the first electrode is annular and is received on the neck and abuts the shoulder surface, and the dilating tip further includes a distal piece received on the neck distally of and abutting the first electrode.

In some examples, the first electrode forms the dilating tip. The medical dilator can include a metallic member that has a first section and a second section, and the first section can join the metallic member to the elongate member while the second section can provide the first electrode and the dilating tip.

In some examples, the electrode is radiopaque. In some examples, the electrode includes platinum-iridium.

In some examples, the electrode has an echogenic profile. In some examples the electrode includes a coil.

According to some aspects, a kit of parts for medical perforation system includes a medical dilator, a sheath, and a perforation device. The medical dilator has an elongate member having a proximal end portion, an opposed distal end portion, and a lumen extending through the elongate member from the proximal end portion to the distal end portion. The medical dilator further has a dilating tip at the distal end portion, and the dilating tip has first end of enlarged cross-sectional area and tapers going in the distal direction to a second end of reduced cross-sectional area. The medical dilator further has at least a first electrode associated with the dilating tip, and an electrical conductor electrically connected to the first electrode and extending proximally from the first electrode to the proximal end portion for electrical connection with an electroanatomical mapping system. The sheath is for receiving the medical dilator. The perforation device is receivable in the lumen.

In some examples, the kit of parts further includes at least a second electrode. The second electrode can be secured to the sheath, or secured to the elongate member, or secured to the perforation device.

In some examples, the sheath has a tip, the medical dilator further includes a second electrode that is electrically connectable to the electroanatomical mapping system, and when the medical dilator is fully inserted into the sheath, the second electrode is proximate the tip of the sheath.

According to some aspects, a medical dilation system includes a medical dilator and an electroanatomical mapping system. The medical dilator includes an elongate member having a proximal end portion, an opposed distal end portion, and a lumen extending through the elongate member from the proximal end portion to the distal end portion. A dilating tip is at the distal end portion. The dilating tip has a first end of enlarged cross-sectional area, and tapers going in the distal direction to a second end of reduced cross-sectional area. At least a first electrode is associated with the dilating tip, and an electrical conductor is electrically connected to the first electrode and extends proximally from the first electrode to the proximal end portion. The electroanatomical mapping system is electrically connectable to the electrical conductor and is configured to receive an electroanatomical mapping signal from the electrode and determine a location of the dilating tip based on the electroanatomical mapping signal.

In some examples, the electroanatomical mapping system is a dielectric open source system.

According to some aspects, a method for medical dilation includes a. advancing a dilating tip of a medical dilator towards a first target anatomical location; b. receiving a first electroanatomical mapping signal from an electrode associated with the dilating tip; and c. based on the first electroanatomical mapping signal, determining a first location of the dilating tip with respect to the first target anatomical location.

In some examples, after step c., the method further includes: d. advancing a perforation device out of the medical dilator, and creating a perforation in the first target anatomical location using the perforation device.

In some examples, the method further includes determining a location of the perforation device with respect to the dilating tip.

In some examples, after step d., the method further includes: e. advancing the electrode and the dilating tip through the perforation, to dilate the perforation.

In some examples, after or during step e., the method further includes: f. receiving a second electroanatomical mapping signal from the electrode, and g. based on the second electroanatomical mapping signal, determining a second location of the dilating tip with respect to the first target anatomical location. In some examples, the first target anatomical location is an atrial septum.

In some examples, the method further includes determining a location of the dilating tip with respect to a left atrial wall.

In some examples, step a. includes positioning the dilator within a sheath and advancing the dilator and the sheath towards the first target anatomical location, and the method further includes determining a location of the dilating tip with respect to a tip of the sheath.

In some examples, the method further includes receiving a second electroanatomical mapping signal from the electrode, and using the second electroanatomical mapping signal to create an anatomical map. The anatomical map can include at least one of a map of a superior vena cava, a map of a right atrium, and a map of pulmonary veins.

### BRIEF DESCRIPTION OF THE DRAWINGS:

The accompanying drawings are for illustrating examples of articles, methods, and apparatuses of the present disclosure and are not intended to be limiting. In the drawings:
Figure 1 is a perspective view of an example surgical perforation system;
Figure 2 is a perspective view of the dilator of the surgical perforation system of Figure 1;
Figure 3A is an enlarged view of the dilating tip of the dilator of Figure 2;
Figure 3B is an end view of the dilating tip of Figure 3A;
Figure 3C is a cross-section taken along line 3C-3C in Figure 3B;
Figure 4A is an enlarged view of another example dilating tip;
Figure 4B is an end view of the dilating tip of Figure 4A;
Figure 4C is a cross-section taken along line 4C-4C in Figure 4B;
Figure 5A is an enlarged view of another example dilating tip;
Figure 5B is a cross-section taken along line 5B-5B in Figure 5A;
Figure 5C is an end view of the dilating tip of Figure 5A;
Figure 5D is a cross-section taken along line 5D-5D in Figure 5C;
Figure 6A is an enlarged view of another example dilating tip;
Figure 6B is an end view of the dilating tip of Figure 6A;
Figure 6C is a cross-section taken along line 6C-6C in Figure 6B;
Figure 7 is a partial perspective view of a sheath, dilator, and perforation device of another example surgical perforation system;
Figure 8 is a schematic view showing a first step of an example method for creation and dilation of a transseptal perforation;
Figure 9 is a schematic view showing a second step of the example method for creation and dilation of a transseptal perforation of Figure 8;
Figure 10 is a schematic view showing a third step of the example method for creation and dilation of a transseptal perforation of Figure 8;
Figure 11 is a schematic view showing a fourth step of the example method for creation and dilation of a transseptal perforation of Figure 8;
Figure 12 is a schematic view showing a fifth step of the example method for creation and dilation of a transseptal perforation of Figure 8;
Figure 13 is a schematic view showing a second step of the example method for creation and dilation of a transseptal perforation of Figure 8;
Figure 14A is a partial side view of another example dilator;
Figure 14B is a cross-section taken along line 14B-14B in Figure 14A; and
Figure 14C is a perspective view of the metallic member of the dilator of Figure 14A.

### DETAILED DESCRIPTION:

Various apparatuses or processes or compositions will be described below to provide an example of an embodiment of the claimed subject matter. No example described below limits any claim and any claim may cover processes or apparatuses or compositions that differ from those described below. The claims are not limited to apparatuses or processes or compositions having all of the features of any one apparatus or process or composition described below or to features common to multiple or all of the apparatuses or processes or compositions described below. It is possible that an apparatus or process or composition described below is not an embodiment of any exclusive right granted by issuance of this patent application. Any subject matter described below and for which an exclusive right is not granted by issuance of this patent application may be the subject matter of another protective instrument, for example, a continuing patent application, and the applicants, inventors or owners do not intend to abandon, disclaim or dedicate to the public any such subject matter by its disclosure in this document.

Generally disclosed herein are medical dilators (also referred to herein simply as "dilators") that can be used for dilation of anatomical apertures, such as surgical perforations. For example, the dilators can be used in transseptal perforation procedures, in which a perforation is created in the atrial septum of the heart, optionally using a radio-frequency perforation device, and then dilated using a dilator. Such procedures can be carried out, for example, to gain access to the left atrium for a medical treatment.

In general, the dilators disclosed herein are configured to allow for non-fluoroscopic visualization and determination of the location of the tip of the dilator (also referred to herein as the "dilating tip") within the body, or of the location of the tip of the dilator with respect to other surgical tools (e.g. with respect to the perforation device or with respect to a sheath in which the dilator is housed). More specifically, the dilators disclosed herein can include at least one electrode associated with the tip thereof. The electrode can be an electroanatomical mapping (EAM) electrode. The EAM electrode can be connected to an EAM system, which can communicate EAM signals to and from the EAM electrode (either directly or via a pad), and based on the EAM signals received from the EAM electrode, can determine a location of the EAM electrode, and thus the tip of the dilator, within the body or with respect to other surgical tools. This can, for example, visualize the dilator tip to allow a user to determine whether the tip is positioned properly with respect to a target tissue, allow a user to confirm that the perforation device is shrouded within the dilator prior to perforation, and/or allow for a user to confirm that the dilating tip is sufficiently spaced from non-target tissues.

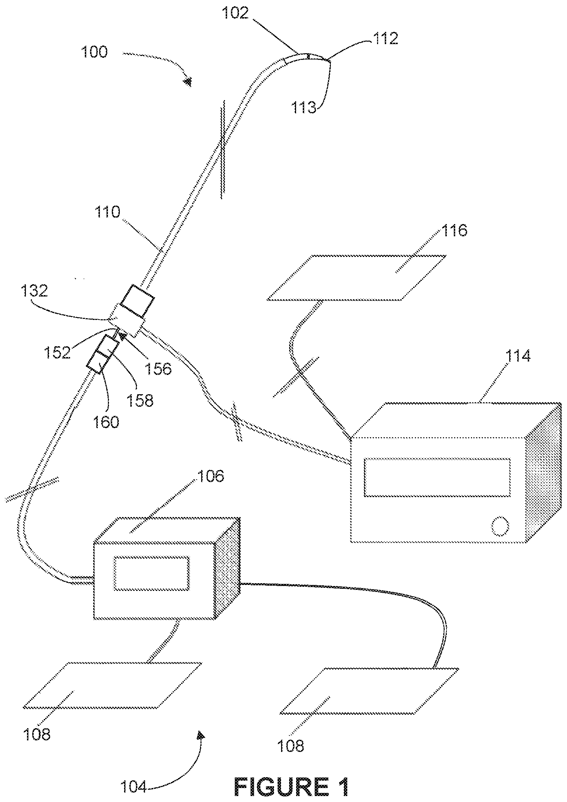

Referring now to Figure 1, an example surgical perforation system 100 is shown. The surgical perforation system 100 includes a dilator 102, an EAM system 104 including an EAM signal generator 106 and a set (e.g. 3 or more) of EAM pads 108 (only two of which are shown in Figure 1), a sheath 110, a radiofrequency (RF) perforation device 112 having a perforation electrode 113 at its distal tip, and an RF generator 114 and grounding pad 116. The sheath 110, RF perforation device 112, RF generator 114, and grounding pad 116 will not be described in detail herein, and can optionally be those sold by Baylis Medical Company, Inc. (Montreal, Canada), for example under the brand names NRG^{®} Transseptal Platform, or SupraCross^{®} Transseptal Platform. Furthermore, in alternative examples, another type of perforation device, such as a mechanical perforation device, can be used instead of an RF perforation device. Optionally, some or all of the parts of the surgical perforation system 100 can be sold or provided together in a kit, either in an assembled state or in an unassembled state.

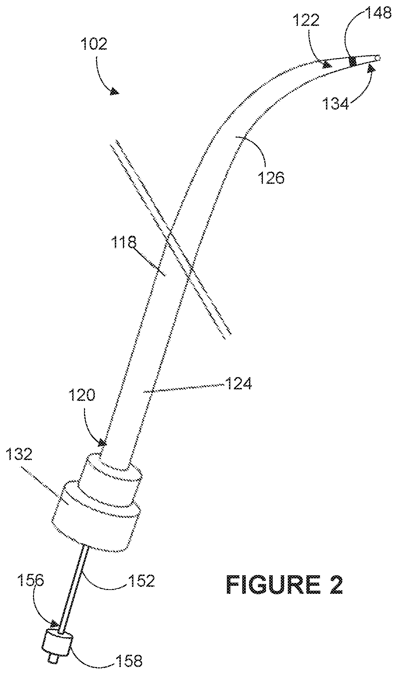

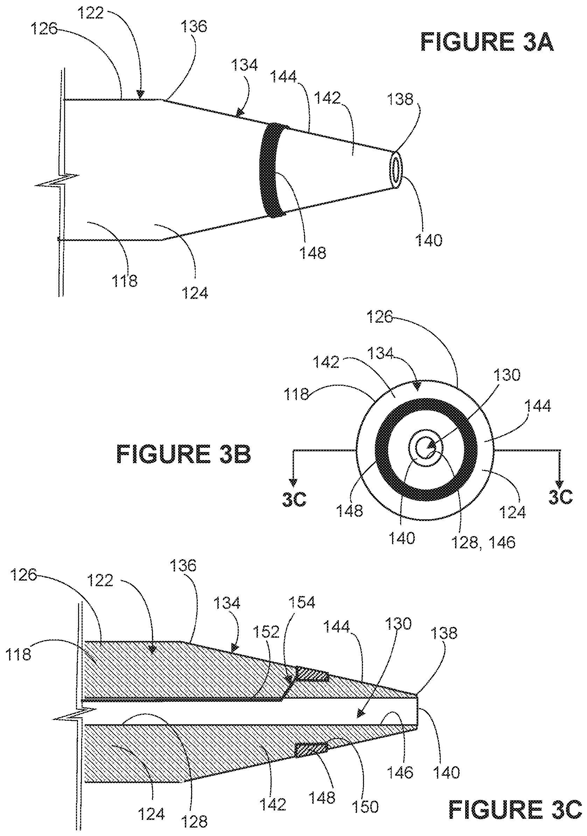

Referring now to Figure 2, the dilator 102 is shown in greater detail. In the example shown, the dilator 102 includes an elongate member 118 having a proximal end portion 120, which in use is generally directed towards a user such as a surgeon, and an opposed distal end portion 122, which in use is generally directed towards a target location in a patient. The elongate member 118 includes a sidewall 124, which extends longitudinally between the proximal end portion 120 and the distal end portion 122, and radially between a circumferential outer surface 126 and a circumferential inner surface 128 (shown in Figures 3B and 3C). The circumferential inner surface 128 defines a lumen 130 (shown in Figures 3B and 3C), which extends through the elongate member 118 from the proximal end portion 120 to the distal end portion 122. In use, the lumen 130 can receive the RF perforation device 112.

The elongate member can be made from various materials, including but not limited to plastics such as high-density polyethylene (HDPE).

Referring still to Figure 2, in the example shown, a handle 132 is mounted to the proximal end portion 120. The handle 132 can include various hubs and/or ports and/or connection points (not shown) for connection to various external devices.

Referring still to Figure 2, the dilator 102 includes a dilating tip 134 at the distal end portion 122. The dilating tip 134 is shown in greater detail in Figures 3A to 3C.

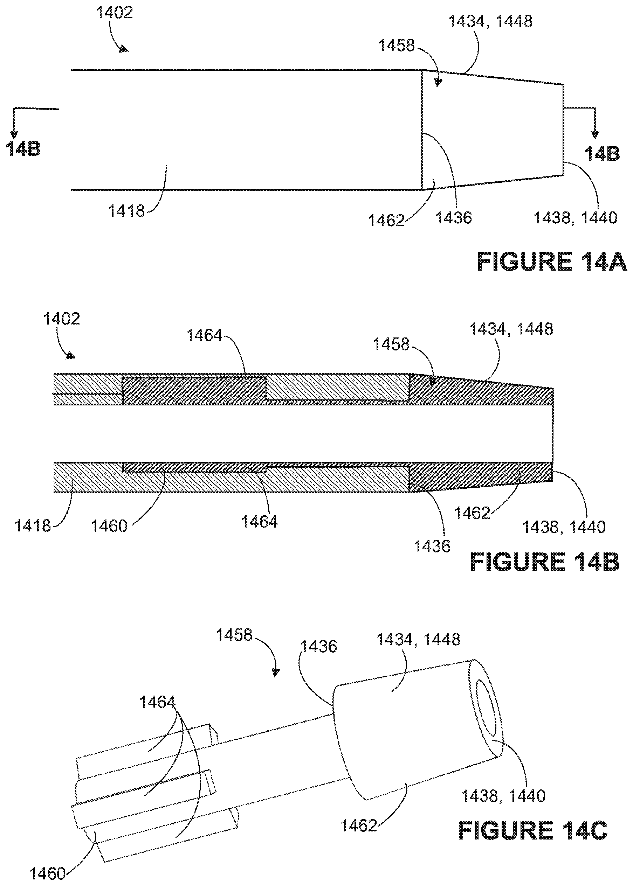

All or a portion of the dilating tip 134 can be integral with the elongate member 118. That is, the distal end portion 122 of the elongate member 118 can include the dilating tip 134, as shown in Figures 3A to 3C. Alternatively, the dilating tip 134 can be a separate piece from the elongate member 118 and can be joined to the distal end portion 122 of the elongate member 118, as described below with respect to Figures 14A to 14C.

In the example shown, the dilating tip 134 includes a first end 136 and a second end 138 that is spaced distally from the first end 136. The dilating tip 134 tapers in cross-sectional area going from the first end 136 to the second end 138, so that the first end 136 has an enlarged cross-sectional area with respect to the second end 138, and the second end 138 has a reduced cross-sectional area with respect to the first end 136. As the dilating tip 134 is passed through an aperture, the enlargement in cross-sectional area dilates the aperture.

In the example shown, the second end 138 of the dilating tip 134 forms a distal end 140 of the dilator 102. In alternative examples (not shown), the dilating tip can be spaced proximally from the distal end of the dilator.

Referring still to Figures 3A to 3C, in the example shown, the dilating tip 134 has a sidewall 142 (also referred to herein as a "tip sidewall"), which extends longitudinally between the first end 136 of the dilating tip 134 and the second end 138 of the dilating tip 134, and radially between an circumferential outer surface 144 of the dilating tip 134 (also referred to herein as a 'tip circumferential outer surface") and an circumferential inner surface 146 of the dilating tip 134 (also referred to herein as a 'tip circumferential inner surface'). The tip sidewall 142, tip circumferential outer surface 144, and tip circumferential inner surface 146 form a part of the sidewall 124 of the elongate member 118, the circumferential outer surface 126 of the elongate member 118, and circumferential inner surface 128 of the elongate member 118, respectively.

Referring still to Figures 3A to 3C, the dilator further includes an EAM electrode 148, which is associated with the dilating tip 134. As described above, the EAM electrode 148 can allow for the location of the dilating tip 134 to be determined, for example the location of the dilating tip 134 within the body, or the location of the dilating tip 134 with respect to other parts of the surgical perforation system 100. The EAM electrode 148 can be, for example, annular, and can be made of or can include stainless steel or platinum-iridium. In some examples, the EAM electrode can additionally be radiopaque, which can allow for visualization of the electrode using fluoroscopy, if desired. In further examples, the EAM electrode can have an echogenic profile, which can allow for visualization of the electrode using ultrasound, if desired. For example, the EAM electrode can include a coil. In some examples, the EAM electrode 148 can be made of a conductive paint.

As mentioned above, the EAM electrode 148 is associated with the dilating tip 134. The term "associated with" indicates that the EAM electrode 148 is positioned to allow for the determination of the location of the dilating tip 134, whether directly (e.g. in cases where the EAM electrode 148 forms all or a part of the dilating tip or in cases where the EAM electrode is mounted directly to the dilating tip 134), or indirectly (e.g. in cases where the EAM electrode 148 is spaced from the dilating tip 134 and where an extrapolation is carried out to determine the location of the dilating tip 134 based on the location of the EAM electrode 148).

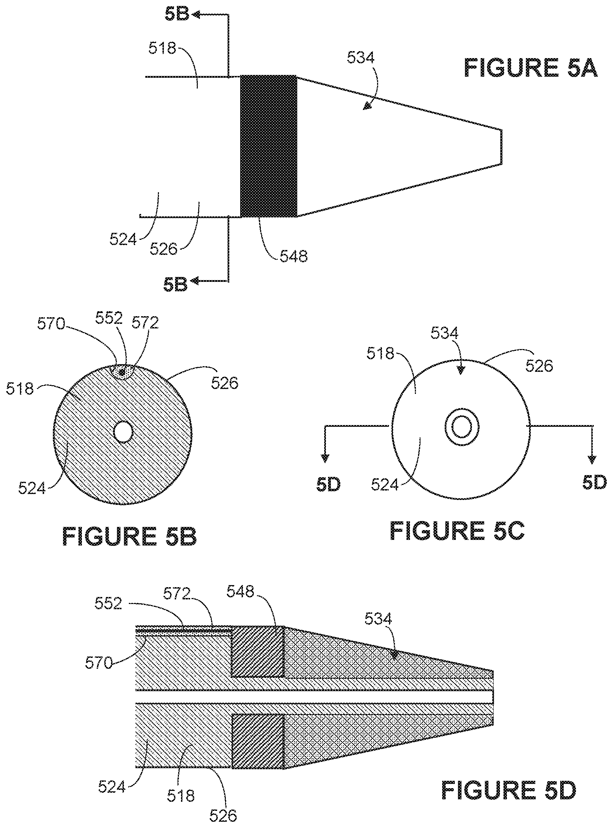

In the example shown, the EAM electrode 148 is annular and extends circumferentially around the dilating tip 134, and is positioned between the first end 136 of the dilating tip 134 and the second end 138 of the dilating tip 134. In alternative examples (e.g. as shown in Figures 5A to 5D), the EAM electrode can be positioned proximal of the dilating tip, or distal of the dilating tip. In such examples, as mentioned above, an extrapolation can be carried out to determine the location of the dilating tip based on the location of the EAM electrode.

Referring to Figure 3C, in the example shown, the circumferential outer surface 144 of the dilating tip 134 has a circumferential groove 150 defined therein, and the EAM electrode 148 is seated in the groove 150. The EAM electrode 148 can be secured in the groove 150 in a variety of ways, such as by gluing, welding, soldering, and/or by friction. Furthermore, in the example shown, the EAM electrode 148 is profiled to match the taper of the dilating tip 134, so that the outer surface of the EAM electrode 148 is flush with the circumferential outer surface 144 of the dilating tip 134. This can be achieved, for example, by swaging. This can allow for a smooth transition as the dilating tip 134 is passed through an aperture.

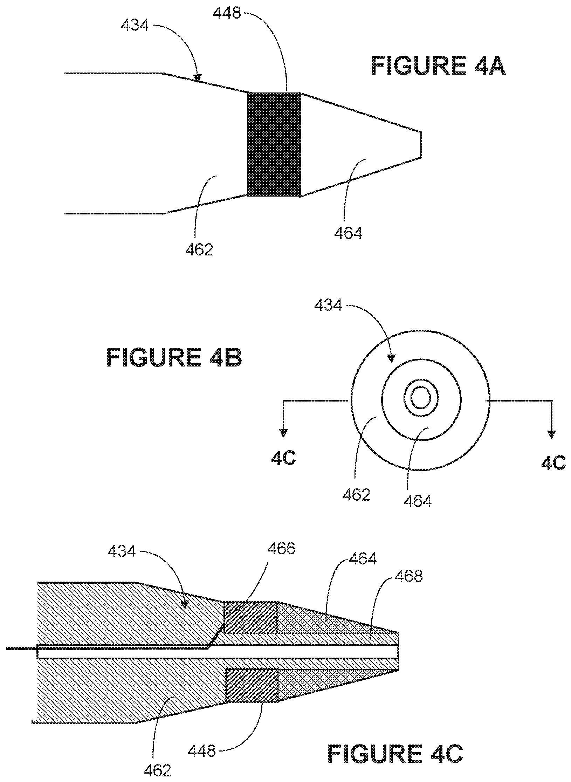

In the example shown, the dilating tip 134 is of a one-piece construction. In alternative examples, as will be described below with reference to Figures 4A to 4C, the dilating tip can be of a multi-piece construction.

Referring still to Figures 3A to 3C, an electrical conductor 152 is connected to the EAM electrode 148, and extends proximally from the EAM electrode 148 towards the proximal end portion 120 (not shown in Figures 3A to 3C) of the elongate member 118, for connection to the EAM signal generator 106 of the EAM system 104 (not shown in Figures 3A to 3C). The electrical conductor 152 is electrically insulated between the EAM electrode 148 and its connection to the EAM signal generator 106, so that electrical signals can be communicated between the EAM electrode 148 and the EAM system 104. For example, the electrical conductor 152 can include a layer of polyimide insulation.

The end of the electrical conductor 152 that is connected to the EAM electrode 148 may be referred to herein as the 'electrode end portion 154' of the electrical conductor 152 (shown in Figure 3C), and the end of the electrical conductor 152 that is connectable to the EAM system 104 may be referred to herein as the 'system end portion 156' of the electrical conductor 152 (shown in Figures 1 and 2). The system end portion 156 of the electrical conductor 152 may be connected or connectable to the EAM signal generator 106 in various ways. In the example shown, a connector 158 is mounted to the system end portion 156. The connector 158 is mateable with a connector 160 of the EAM signal generator 106. Alternatively, clips (e.g. alligator clips) may be used to connect the system end portion of the electrical conductor to the EAM system (not shown).

Referring still to Figures 3A to 3C, in the example shown, the electrical conductor 152 extends from the EAM electrode 148, through the tip sidewall 142, and into the lumen 130. The electrical conductor 152 then extends through the lumen 130 to the proximal end portion 120 of the elongate member 118. In alternative examples, as will be described below, the electrical conductor can be embedded within the sidewall of the elongate member.

As mentioned above, in the example shown, the EAM system 104 includes the EAM signal generator 106 and a pair of EAM pads 108. Such systems are commercially available, for example under the brand names ENSITE PRECISION^{™} and CARTO^{®}, and are not described in detail herein. Briefly, by routing electrical signals from the EAM signal generator 106 to the EAM pads 108, from the EAM pads 108 to the EAM electrode 148, and from the EAM electrode 148 back to the EAM signal generator 106 (or in the reverse order - i.e. from the EAM signal generator 106 to the EAM electrode 148, from the EAM electrode 148 to the EAM pads 108, and from the EAM pads 108 back to the EAM signal generator 106), the EAM electrode 148 may be visualized, and thus the location of the dilating tip 134, can be determined. In some examples, the EAM system 104 can be a di-electric open source EAM system (e.g. one available under the brand name KODEX-EPD). In addition to allowing for the determination of a location of the dilating tip of the dilator, such as system can allow for the dilator to be used for anatomical mapping (e.g. to map the geometry of heart chambers) without necessarily contacting heart tissue, as described in further detail below.

In the example shown, the perforation electrode 113 of the RF perforation device 112 can also be used as an additional EAM electrode. That is, together with the EAM electrode 148 of the dilator 102, the perforation electrode 113 of the RF perforation device 112 can be electrically connected to the EAM system 104, so that its location can be determined by the EAM system 104.

Referring now to Figures 4A to 4C, an alternative example of a dilating tip is shown. In Figure 4, features that are like those of Figures 1 to 3 will be referred to with like reference numerals, incremented by 300. The dilating tip 434 of Figure 4 is similar to the dilating tip 134 of Figures 1 to 3; however, the dilating tip 434 is of a multi-piece construction. Specifically, in the example shown, the dilating tip 434 includes a proximal piece 462, and a distal piece 464. The proximal piece 462 is stepped to define a distal-facing shoulder surface 466, and has a neck 468 extending distally from the shoulder surface 466. The EAM electrode 448 is annular and is received on the neck 468 and abuts the shoulder surface 466. The distal piece 464 is received on the neck 468 distally of the EAM electrode 448 and abuts the EAM electrode 448. The proximal piece 462, EAM electrode 448, and distal piece 464 can be secured together in a variety of ways, such as by adhering and/or friction.

Referring now to Figures 5A to 5D, another alternative example of a dilating tip is shown. In Figure 5, features that are like those of Figures 1 to 3 will be referred to with like reference numerals, incremented by 400. The dilating tip 534 of Figure 5 is similar to the dilating tip 134 of Figures 1 to 3; however, the electrical conductor 552 is embedded in the sidewall 524 of the elongate member 518. Specifically, the circumferential outer surface 526 of the elongate member 518 has a longitudinal groove 570 defined therein. The groove 570 extends from the EAM electrode 548 to the proximal end portion (not shown) of the elongate member 518. The electrical conductor 552 is seated in the groove 570, and a strip of material 572 (e.g. plastic or glue) fills the groove 570 over the electrical conductor 552.

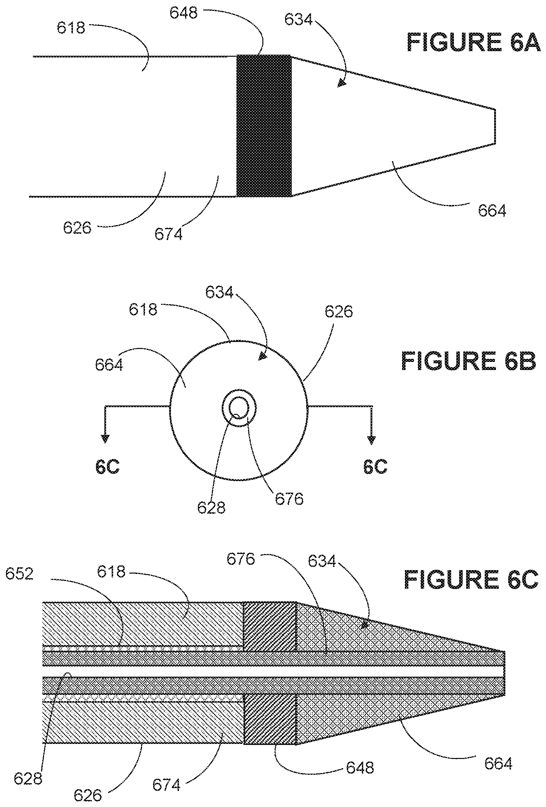

Referring now to Figures 6A to 6C, another alternative example of a dilating tip is shown. In Figure 6, features that are like those of Figures 1 to 3 will be referred to with like reference numerals, incremented by 500. The dilating tip 634 of Figure 6 is similar to the dilator of Figures 1 to 3; however, the elongate member 618 includes an outer tube 674, which defines the circumferential outer surface 626, and an inner liner 676 within the outer tube 674, which defines the circumferential inner surface 628. The inner liner 676 can be, for example, a polyimide or polytetrafluoroethylene liner, and the outer tube 674 can be made of a plastic such as HDPE.

In the example of Figure 6, the electrical conductor 652 is defined by a tubular braid of metallic wires, which is positioned between the outer tube 674 and inner liner 676.

Optionally, in order to fabricate the dilator of Figure 6, the outer tube 674, electrical conductor 652, EAM electrode 648, and inner liner 676 can first be assembled together, and the EAM electrode 648 can be swaged to form an electrical connection between the EAM electrode 648 and the electrical conductor 652. Then, the material of the outer tube 674 can be re-flowed (e.g. by the application of heat) to join the outer tube 674, electrical conductor 652, and inner liner 676. A distal piece 664 of the dilating tip 634 can then be joined to the assembly. The system end (not shown) of the electrical conductor 652 can then be exposed for connection to the EAM system 104, optionally by skiving.

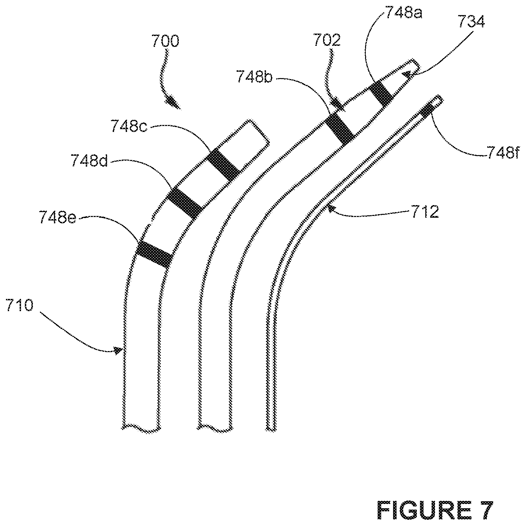

Referring now to Figure 7, another example of a surgical perforation system is shown. In Figure 7, features that are like those of Figures 1 will be referred to with like reference numerals, incremented by 600. In Figure 7, only the dilator 702, sheath 710, and RF perforation device 712 of the system 700 are shown; the remaining parts of the system 700 can be the same as or similar to the parts shown in Figure 1. The system 700 of Figure 7 includes additional EAM electrodes. Specifically, the system 700 includes a first EAM electrode 748a associated with the dilating tip, as described above with respect to Figures 1 to 3. Additionally, the system includes a second EAM electrode 748b on the dilator 702 and spaced from the first EAM electrode 748a; third 748c, fourth 748d, and fifth 748e EAM electrodes on the sheath 710; and a sixth EAM electrode 748f on the RF perforation device 712. The second through sixth EAM electrodes (748b - 748f) are connectable to the EAM signal generator via additional electrical conductors (not shown). The use of additional EAM electrodes can allow for additional location data to be determined. For example, the location of the sheath 710, or the location dilating tip 734 with respect to the sheath 710, can be determined. Additionally, by providing additional electrodes, the orientation of the sheath or dilator may be determined. For example, providing at least two electrodes on each of the sheath and dilator allows the determination of the direction in which the devices are oriented.

In another example (not shown), a dilator can be similar to the dilator 702 of Figure 7; however the second EAM electrode can be positioned so that when the dilator is fully inserted into the sheath, the second EAM electrode is proximate the tip of the sheath (e.g. flush with or close to flush with the tip of the sheath). In such examples, even if the sheath itself does not include any EAM electrodes, the second EAM electrode of the dilator can be used to determine a location of the tip of the sheath when the dilator is fully inserted into the sheath. For example, the second EAM electrode of the dilator can be used to determine whether tip of the sheath has entered the left atrium during a transseptal perforation procedure.

Referring now to Figures 14A to 14C, another example of a dilator is shown. In Figures 14A to 14C, features that are like those of Figures 1 to 3 will be referred to with like reference numerals, incremented by 1300. In the dilator 1402, the dilating tip 1434 is similar to the dilating tip 134 of Figures 1 to 3; however, the EAM electrode 1448 forms the dilating tip 1434. That is, a metallic member 1458 (shown in isolation in Figure 14C) is provided that includes a first section 1460 and a second section 1462. The first section 1460 secures the metallic member 1458 to the elongate member 1418, while the second section 1462 serves as the EAM electrode 1448 and also forms the dilating tip 1434. The first section 1460 includes ribs 1464 that are embedded in the elongate member 1418, to secure the metallic member 1458 to the elongate member 1418. The second section 1462 extends distally from the first section 1460, and tapers in cross-sectional area going from a first end 1436 thereof to a second end thereof 1438, to form the dilating tip 1434. In this example, the EAM electrode 1448 forms the distal end 1440 of the dilator 1402, and therefore can allow for tissue contact with the EAM electrode.

In a further alternative example of a dilator (not shown), the EAM electrode can be removable from the elongate member. For example, the elongate member of the dilator can be a standard dilator (e.g. one known in the art). The EAM electrode, connected to the electrical conductor, can be separate from the elongate member. For example, the EAM electrode can be secured to the perforation device. The EAM electrode can be advanced through the lumen of the elongate member, until the EAM electrode is at the distal end of the dilator. The assembly can be calibrated so that the extent to which the EAM electrode should be advanced to reach the distal end is known.

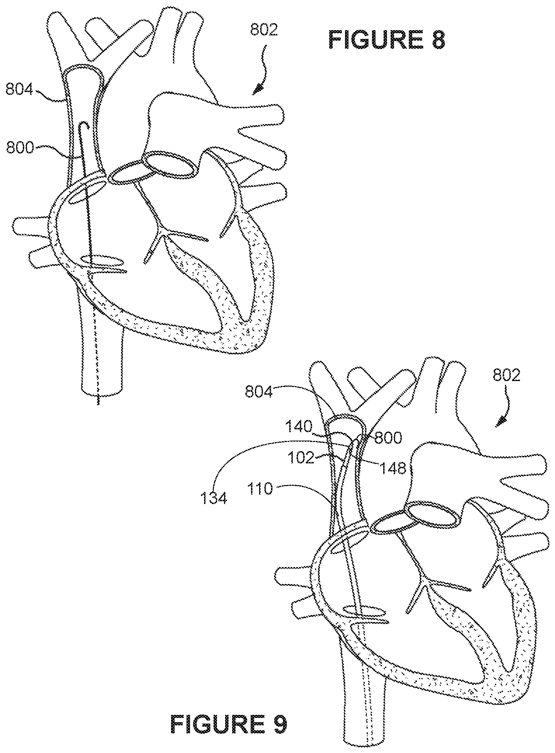

Referring now to Figures 8 to 13, a method for medical dilation, specifically for creation and dilation of a transseptal perforation, will be described. As will be described in more detail, at various points during the method, the EAM electrode and EAM system can be engaged to determine the location of the dilating tip of the dilator - i.e. EAM signals can be received from the EAM electrode of the dilator, and based on the EAM signals, the location of the dilating tip of the dilator can be determined, and optionally mapped and tracked. This can enhance safety of the procedure. The method will be described with reference to the system 100 and dilator 102 as shown in Figures 1 to 3; however, the method is not limited to being carried out with system 100 and dilator 102, and the system 100 and dilator 102 are not limited to use according to the described method.

Referring to Figure 8, a guidewire 800 can be advanced via the femoral vein towards the heart 802, and "parked" in the superior vena cava (SVC) 804.

Referring to Figure 9, with the dilator 102 in the sheath 110, and with the dilating tip 134 extending proud of the sheath 110, the dilator 102 and sheath 110 can be advanced over the guidewire 800 towards the SVC 804. The guidewire 800 can then be removed, and the RF perforation device 112 (not shown in Figure 9) can be advanced through the dilator 102 until the perforation electrode 113 (not shown in Figure 9) of the RF perforation device 112 is just shy of the distal end 140 of the dilator 102.

As mentioned above, in addition to the EAM electrode 148 of the dilator 102 being connected to the EAM system 104 (not shown in Figures 8 to 13), the perforation electrode 113 of the RF perforation device 112 can be connected to the EAM system 104 and can serve as an additional EAM electrode. After the RF perforation device 112 has been advanced through the dilator 102 and the perforation electrode 113 is exposed from the dilator 102 or the distal tip of the perforation device 112 is flush with the distal tip of the dilator 102, the positioning of the perforation device 112 can be confirmed using the EAM system 104. Specifically, the EAM system 104 can be engaged, and based on the EAM signal received from the EAM electrode 148 and the perforation electrode 113, the location of the perforation electrode 113 with respect to the dilating tip 134 can be determined. For example, if the EAM system shows that the perforation electrode 113 is proud of the dilating tip 134, it can be determined that the perforation electrode 113 has been advanced too far into the dilator 102. Alternatively, if the perforation electrode 113 cannot be detected by the EAM system, it can be concluded that the perforation electrode 113 is shrouded within the dilating tip 134, and therefore correctly positioned. Additionally, by providing both a perforation electrode 113 and an EAM electrode 148, the relative positioning between the two may be mapped to allow determination of the orientation of the combined assembly.

In some examples, the system 100 can further be configured to provide an alert if the perforation electrode 113 advances distal of the distal end 140 of the dilator 102.

Optionally, at this point, if anatomical data is desired, the user can refer to CT or MRI data.

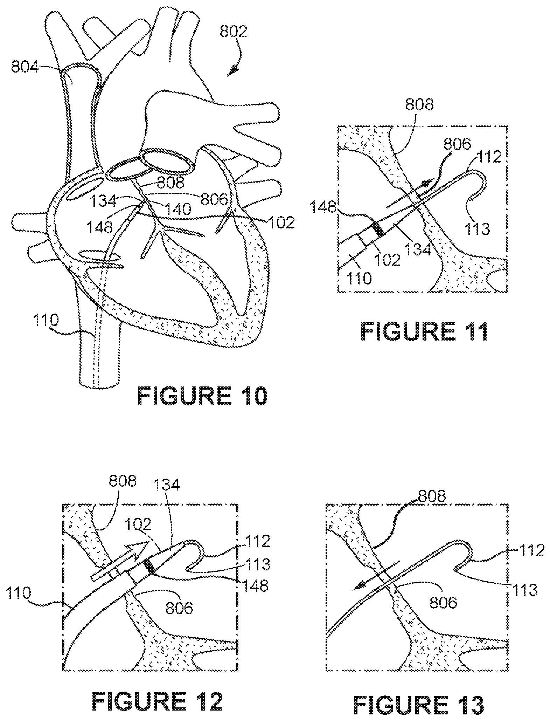

Referring now to Figure 10, with the EAM electrode 148 and EAM system 104 engaged to track the location of the dilating tip 134 and the perforation electrode 113 (not shown in Figure 10), the sheath 110, dilator 102, and perforation device 112 can be advanced towards a target anatomical location to position the dilating tip 134 at the target location. The target anatomical location can be, for example, the fossa ovalis 806 of the atrial septum 808. The EAM electrode 148 and EAM system 104 can be used to confirm the positioning of the dilating tip 134 against the fossa ovalis 806, and also to confirm that the perforation electrode 113 is flush with the distal end 140 of the dilator 102.

Referring to Figure 11, the perforation device 112 can then be engaged and advanced out of the dilator 102, to create a perforation in the atrial septum 808.

Referring to Figure 12, the dilating tip 134 can then be advanced through the perforation, to dilate the perforation. Specifically, the dilating tip 134, together with the EAM electrode 148, can be advanced through the perforation. Prior to, during and/or after advancement of the dilating tip 134 and EAM electrode 148, the EAM electrode 148 and EAM system 104 can be engaged to determine the location of the dilating tip 134. This can help to ensure that the perforation is sufficiently dilated, while also helping to ensure that the dilating tip 134 does not contact and thereby damage non-target tissues (e.g. the location of the dilating tip with respect to the left atrial wall can be visualized).

Following dilation of the perforation, various procedures can be carried out. At the desired time, as shown in Figure 13, the dilator 102 and sheath 110 can be withdrawn from the heart 802. Optionally, during withdrawal, the EAM electrode 148 and EAM system 104 can be engaged, to determine the location of the dilating tip 134.

Optionally, throughout the method, anatomical mapping can be carried out using the dilator 1402 and the EAM system 104. That is, as mentioned above, if the EAM system 104 is a dielectric open source EAM system, the dilator 102 and EAM system 104 can be used for cardiac mapping, without necessarily contacting any cardiac tissue. For example, during advancement of the dilator 102, when the dilator 102 is in the inferior vena cava, the EAM system 104 can be engaged (i.e. a electroanatomical signal can be received from the EAM electrode 148) to map the SVC 804. For further example, when the dilator 102 is in the right atrium 808, the EAM system 104 can be engaged to map the right atrium 808. For further example, after the dilator 102 crosses the atrial septum 808, the EAM system 104 can be engaged to map the pulmonary veins. This can be facilitated by rotating the dilator 102. In such examples, as the dilator 102 advances towards a location that is to be mapped, more detail and increasing resolution may be achieved. Furthermore, in examples where the EAM electrode is at the distal end of the dilator, the EAM system may be able to provide an alert when tissue is contacted by the distal end of the dilator.

## Claims

1. A medical dilator (102, 1402), comprising:
an elongate member (118, 1418) having a proximal end portion (120), an opposed distal end portion (122), and a lumen (130) extending through the elongate member (118, 1418) from the proximal end portion (120) to the distal end portion (122);
a dilating tip (134, 434, 1434) at the distal end portion (122), the dilating tip (134, 434, 1434) having first end (136) of enlarged cross-sectional area and tapering going in the distal direction to a second end (138) of reduced cross-sectional area;
at least a first electrode (148, 448, 1448) associated with the dilating tip (134, 434, 1434); and
an electrical conductor (152, 652) electrically connected to the first electrode (148, 448, 1448) and extending proximally from the first electrode (148, 448, 1448) towards the proximal end portion (120) for electrical connection with an electroanatomical mapping system (104);
**characterised in that**:
the dilating tip (134, 434, 1434) comprises a proximal piece (462) having a distal-facing shoulder surface (466) and a neck (468) extending distally from the shoulder surface (466);
the first electrode (148, 448, 1448) is annular and is received on the neck (468) and abuts the shoulder surface (466); and
the dilating tip (134, 434, 1434) further comprises a distal piece (464) received on the neck (468) distally of and abutting the first electrode (148, 448, 1448).

2. The medical dilator (102, 1402) of claim 1, wherein the first electrode (148, 448, 1448) is positioned between the first end (136) of the dilating tip (134, 434, 1434) and the second end (138) of the dilating tip (134, 434, 1434).

3. The medical dilator (102, 1402) of claim 1, wherein the first electrode (148, 448, 1448) is positioned proximal of the first end (136) of the dilating tip (134, 434, 1434).

4. The medical dilator (102, 1402) of any one of claims 1 to 3, wherein the dilating tip (134, 434, 1434) has a tip circumferential outer surface (144) having a circumferential groove (150) defined therein, and the first electrode (148, 448, 1448) is annular and is seated in the groove (150).

5. The medical dilator (102, 1402) of claim 4, wherein the first electrode (148, 448, 1448) has an electrode outer surface, and the electrode outer surface is flush with the tip circumferential outer surface (144).

6. The medical dilator (102, 1402) of any one of claims 1 to 3, wherein the dilating tip (134, 434, 1434) has a tip circumferential outer surface (144), a tip circumferential inner surface (146), and a tip sidewall (142) extending between the tip circumferential inner surface (146) and the tip circumferential outer surface (144), and the electrical conductor (152, 652) extends from the first electrode (148, 448, 1448) through the tip sidewall (142) and into the lumen (130).

7. The medical dilator (102, 1402) of any one of claims 1 to 3, wherein the elongate member (118, 1418) has a circumferential outer surface (126), a circumferential inner surface (128), and a sidewall (124) extending along the length of the elongate member (118, 1418) between the circumferential inner surface (128) and the circumferential outer surface (126), and the electrical conductor (152, 652) is embedded in the sidewall (124) and extends from the first electrode (148, 448, 1448) to the proximal end portion (120).

8. The medical dilator (102, 1402) of claim 7, wherein the circumferential outer surface (126) has a longitudinal groove (570) defined therein and extending from the first electrode (148, 448, 1448) to the proximal end portion (120), and wherein the electrical conductor (152, 652) is seated in the longitudinal groove (570).

9. The medical dilator (102, 1402) of claim 7, wherein the elongate member (118, 1418) comprises an outer tube (674) defining the circumferential outer surface (126), and an inner liner (676) within the outer tube (674) and defining the circumferential inner surface (128), and wherein the electrical conductor (152, 652) is a tubular braid and is positioned between the outer tube (674) and the inner liner (676).

10. The medical dilator (102, 1402) of any one of claims 1 to 9, wherein the first electrode (148, 448, 1448) is removable from the elongate member (118, 1418).

11. The medical dilator (102, 1402) of any one of claims 1 to 10, further comprising a second electrode (748b) mounted to the elongate member (118, 1418) and spaced from the first electrode (148, 448, 1448).

12. The medical dilator (102, 1402) of claim 1, wherein the first electrode (148, 448, 1448) forms the dilating tip (134, 434, 1434).

13. The medical dilator (102, 1402) of claim 12, further comprising a metallic member (1458) having a first section (1460) and a second section (1462), wherein the first section (1460) joins the metallic member (1458) to the elongate member (118, 1418), and the second section (1462) provides the first electrode (148, 448, 1448) and the dilating tip (134, 434, 1434).

14. The medical dilator (102, 1402) of any one of claims 1 to 13, wherein the first electrode (148, 448, 1448) is radiopaque or includes an echogenic profile.

## Patentansprüche

1. Medizinischer Dilatator (102, 1402), umfassend:
ein längliches Element (118, 1418), das einen proximalen Endteil (120), einen gegenüberliegenden distalen Endteil (122) und ein Lumen (130) aufweist, das sich durch das längliche Element (118, 1418) von dem proximalen Endteil (120) zu dem distalen Endteil (122) erstreckt;
eine Dilatationsspitze (134, 434, 1434) an dem distalen Endteil (122), wobei die Dilatationsspitze (134, 434, 1434) ein erstes Ende (136) mit vergrößerter Querschnittsfläche aufweist und sich in distaler Richtung zu einem zweiten Ende (138) mit reduzierter Querschnittsfläche verjüngt;
mindestens eine erste Elektrode (148, 448, 1448), die mit der Dilatationsspitze (134, 434, 1434) assoziiert ist; und
einen elektrischen Leiter (152, 652), der elektrisch mit der ersten Elektrode (148, 448, 1448) verbunden ist und sich proximal von der ersten Elektrode (148, 448, 1448) in Richtung des proximalen Endteils (120) erstreckt zur elektrischen Verbindung mit einem elektroanatomischen Zuordnungssystem (104);
**dadurch gekennzeichnet, dass**
die Dilatationsspitze (134, 434, 1434) ein proximales Teil (462) umfasst, das eine distal ausgerichtete Schulterfläche (466) und einen Hals (468) aufweist, der sich distal von der Schulterfläche (466) erstreckt;
die erste Elektrode (148, 448, 1448) ringförmig ist und an dem Hals (468) aufgenommen ist und an der Schulterfläche (466) anliegt; und
die Dilatationsspitze (134, 434, 1434) des Weiteren ein distales Teil (464) umfasst, das an dem Hals (468) distal von der ersten Elektrode (148, 448, 1448) aufgenommen ist und an dieser anliegt.

2. Medizinischer Dilatator (102, 1402) nach Anspruch 1, wobei die erste Elektrode (148, 448, 1448) zwischen dem ersten Ende (136) der Dilatationsspitze (134, 434, 1434) und dem zweiten Ende (138) der Dilatationsspitze (134, 434, 1434) positioniert ist.

3. Medizinischer Dilatator (102, 1402) nach Anspruch 1, wobei die erste Elektrode (148, 448, 1448) proximal zu dem ersten Ende (136) der Dilatationsspitze (134, 434, 1434) positioniert ist.

4. Medizinischer Dilatator (102, 1402) nach einem der Ansprüche 1 bis 3, wobei die Dilatationsspitze (134, 434, 1434) eine spitzenumlaufende Außenfläche (144) aufweist, in der eine Umfangsnut (150) definiert ist, und die erste Elektrode (148, 448, 1448) ringförmig ist und in der Nut (150) sitzt.

5. Medizinischer Dilatator (102, 1402) nach Anspruch 4, wobei die erste Elektrode (148, 448, 1448) eine Elektrodenaußenfläche aufweist und die Elektrodenaußenfläche bündig mit der spitzenumlaufenden Außenfläche (144) der Spitze abschließt.

6. Medizinischer Dilatator (102, 1402) nach einem der Ansprüche 1 bis 3, wobei die Dilatationsspitze (134, 434, 1434) eine spitzenumlaufende Außenfläche (144), eine spitzenumlaufende Innenfläche (146) und eine Seitenwand (142) aufweist, die sich zwischen der spitzenumlaufenden Innenfläche (146) und der spitzenumlaufenden Außenfläche (144) erstreckt, und sich der elektrische Leiter (152, 652) von der ersten Elektrode (148, 448, 1448) durch die Spitzenseitenwand (142) und in das Lumen (130) erstreckt.

7. Medizinischer Dilatator (102, 1402) nach einem der Ansprüche 1 bis 3, wobei das längliche Element (118, 1418) eine umlaufende Außenfläche (126), eine umlaufende Innenfläche (128) und eine Seitenwand (124) aufweist, die sich entlang der Länge des länglichen Elements (118, 1418) zwischen der umlaufenden Innenfläche (128) und der umlaufenden Außenfläche (126) erstreckt, und der elektrische Leiter (152, 652) in die Seitenwand (124) eingebettet ist und sich von der ersten Elektrode (148, 448, 1448) zu dem proximalen Endteil (120) erstreckt.

8. Medizinischer Dilatator (102, 1402) nach Anspruch 7, wobei die umlaufende Außenfläche (126) eine darin definierte Längsnut (570) aufweist, die sich von der ersten Elektrode (148, 448, 1448) zu dem proximalen Endteil (120) erstreckt, und wobei der elektrische Leiter (152, 652) in der Längsnut (570) sitzt.

9. Medizinischer Dilatator (102, 1402) nach Anspruch 7, wobei das längliche Element (118, 1418) ein Außenrohr (674) umfasst, das die umlaufende Außenfläche (126) bildet, und eine Innenauskleidung (676) innerhalb des Außenrohrs (674), die die umlaufende Innenfläche (128) definiert, und wobei der elektrische Leiter (152, 652) ein rohrförmiges Geflecht ist und zwischen dem Außenrohr (674) und der Innenauskleidung (676) positioniert ist.

10. Medizinischer Dilatator (102, 1402) nach einem der Ansprüche 1 bis 9, wobei die erste Elektrode (148, 448, 1448) von dem länglichen Element (118, 1418) abnehmbar ist.

11. Medizinischer Dilatator (102, 1402) nach einem der Ansprüche 1 bis 10, des Weiteren umfassend eine zweite Elektrode (748b), die an dem länglichen Element (118, 1418) montiert und von der ersten Elektrode (148, 448, 1448) beabstandet ist.

12. Medizinischer Dilatator (102, 1402) nach Anspruch 1, wobei die erste Elektrode (148, 448, 1448) die Dilatationsspitze (134, 434, 1434) bildet.

13. Medizinischer Dilatator (102, 1402) nach Anspruch 12, des Weiteren umfassend ein metallisches Element (1458), das eine erste Sektion (1460) und eine zweite Sektion (1462) aufweist, wobei die erste Sektion (1460) das metallische Element (1458) mit dem länglichen Element (118, 1418) verbindet, und die zweite Sektion (1462) die erste Elektrode (148, 448, 1448) und die Dilatationsspitze (134, 434, 1434) bereitstellt.

14. Medizinischer Dilatator (102, 1402) nach einem der Ansprüche 1 bis 13, wobei die erste Elektrode (148, 448, 1448) röntgendicht ist und ein echogenes Profil beinhaltet.

## Revendications

1. Dilatateur médical (102, 1402), comprenant :
un élément allongé (118, 1418) qui comporte une partie d'extrémité proximale (120), une partie d'extrémité distale opposée (122) et une lumière (130) qui est étendue à travers l'élément allongé (118, 1418) depuis la partie d'extrémité proximale (120) jusqu'à la partie d'extrémité distale (122) ;
un embout de dilatation (134, 434, 1434) au niveau de la partie d'extrémité distale (122), l'embout de dilatation (134, 434, 1434) comportant une première extrémité (136) qui présente une section en coupe transversale agrandie et qui est effilée au fur et à mesure de la progression dans la direction distale jusqu'à une seconde extrémité (138) qui présente une section en coupe transversale réduite ;
au moins une première électrode (148, 448, 1448) associée à l'embout de dilatation (134, 434, 1434) ; et
un conducteur électrique (152, 652) connecté électriquement à la première électrode (148, 448, 1448) et étendu de façon proximale depuis la première électrode (148, 448, 1448) en direction de la partie d'extrémité proximale (120) dans un but de connexion électrique à un système de cartographie électro-anatomique (104) ;
**caractérisé en ce que** :
l'embout de dilatation (134, 434, 1434) comprend une pièce proximale (462) qui comporte une surface d'épaulement (466) orientée de façon distale et un col (468) étendu de façon distale depuis la surface d'épaulement (466) ;
la première électrode (148, 448, 1448) est annulaire, est reçue sur le col (468) et vient en butée contre la surface d'épaulement (466) ; et
l'embout de dilatation (134, 434, 1434) comprend en outre une pièce distale (464) reçue sur le col (468) de façon distale par rapport à la première électrode (148, 448, 1448) et venant en butée contre celle-ci.

2. Dilatateur médical (102, 1402) selon la revendication 1, dans lequel la première électrode (148, 448, 1448) est positionnée entre la première extrémité (136) de l'embout de dilatation (134, 434, 1434) et la seconde extrémité (138) de l'embout de dilatation (134, 434, 1434).

3. Dilatateur médical (102, 1402) selon la revendication 1, dans lequel la première électrode (148, 448, 1448) est positionnée de façon proximale par rapport à la première extrémité (136) de l'embout de dilatation (134, 434, 1434).

4. Dilatateur médical (102, 1402) selon l'une quelconque des revendications 1 à 3, dans lequel l'embout de dilatation (134, 434, 1434) comporte une surface externe circonférentielle d'embout (144) qui comporte une gorge circonférentielle (150) formée en son sein, et la première électrode (148, 448, 1448) est annulaire et est logée dans la gorge (150).

5. Dilatateur médical (102, 1402) selon la revendication 4, dans lequel la première électrode (148, 448, 1448) comporte une surface externe d'électrode, et la surface externe d'électrode affleure la surface externe circonférentielle d'embout (144).

6. Dilatateur médical (102, 1402) selon l'une quelconque des revendications 1 à 3, dans lequel l'embout de dilatation (134, 434, 1434) comporte une surface externe circonférentielle d'embout (144), une surface interne circonférentielle d'embout (146) et une paroi latérale d'embout (142) qui est étendue entre la surface interne circonférentielle d'embout (146) et la surface externe circonférentielle d'embout (144), et le conducteur électrique (152, 652) est étendu depuis la première électrode (148, 448, 1448) à travers la paroi latérale d'embout (142) et à l'intérieur de la lumière (130).

7. Dilatateur médical (102, 1402) selon l'une quelconque des revendications 1 à 3, dans lequel l'élément allongé (118, 1418) comporte une surface externe circonférentielle (126), une surface interne circonférentielle (128) et une paroi latérale (124) qui est étendue suivant la longueur de l'élément allongé (118, 1418) entre la surface interne circonférentielle (128) et la surface externe circonférentielle (126), et le conducteur électrique (152, 652) est intégré dans la paroi latérale (124) et est étendu depuis la première électrode (148, 448, 1448) jusqu'à la partie d'extrémité proximale (120).

8. Dilatateur médical (102, 1402) selon la revendication 7, dans lequel la surface externe circonférentielle (126) comporte une gorge longitudinale (570) qui est formée en son sein et qui est étendue depuis la première électrode (148, 448, 1448) jusqu'à la partie d'extrémité proximale (120), et dans lequel le conducteur électrique (152, 652) est logé dans la gorge longitudinale (570).

9. Dilatateur médical (102, 1402) selon la revendication 7, dans lequel l'élément allongé (118, 1418) comprend un tube externe (674) qui définit la surface externe circonférentielle (126) et un revêtement interne (676) qui est disposé à l'intérieur du tube externe (674) et qui définit la surface interne circonférentielle (128), et dans lequel le conducteur électrique (152, 652) est une tresse tubulaire et est positionné entre le tube externe (674) et le revêtement interne (676).

10. Dilatateur médical (102, 1402) selon l'une quelconque des revendications 1 à 9, dans lequel la première électrode (148, 448, 1448) est amovible par rapport à l'élément allongé (118, 1418).

11. Dilatateur médical (102, 1402) selon l'une quelconque des revendications 1 à 10, comprenant en outre une seconde électrode (748b) montée sur l'élément allongé (118, 1418) et espacée de la première électrode (148, 448, 1448).

12. Dilatateur médical (102, 1402) selon la revendication 1, dans lequel la première électrode (148, 448, 1448) forme l'embout de dilatation (134, 434, 1434).

13. Dilatateur médical (102, 1402) selon la revendication 12, comprenant en outre un élément métallique (1458) qui comporte une première section (1460) et une seconde section (1462), dans lequel la première section (1460) relie l'élément métallique (1458) à l'élément allongé (118, 1418) et la seconde section (1462) constitue la première électrode (148, 448, 1448) et l'embout de dilatation (134, 434, 1434).

14. Dilatateur médical (102, 1402) selon l'une quelconque des revendications 1 à 13, dans lequel la première électrode (148, 448, 1448) est radio-opaque ou comprend un profil échogène.
